# EUROPÄISCHE PATENTSCHRIFT

(11) **EP 1 067 995 B1**
(45) Veröffentlichungstag und Bekanntmachung des Hinweises auf die Patenterteilung: **09.04.2003**
(21) Anmeldenummer: 99942579.6
(22) Anmeldetag: 23.02.1999
(51) Int. Cl.: B01D 7/02, C07C 51/573

(54) **VERFAHREN ZUM ABTRENNEN VON DAMPFFÖRMIGEM PHTHALSÄUREANHYDRID AUS EINEM GASSTROM**
METHOD FOR SEPARATING VAPOROUS PHTHALIC ACID ANHYDRIDE FROM A GAS STREAM
PROCEDE DE SEPARATION DE VAPEURS D'ANHYDRIDE D'ACIDE PHTALIQUE D'UN FLUX GAZEUX

(30) Priorität: 26.03.1998 DE 19813286
(43) Veröffentlichungstag der Anmeldung: 17.01.2001
(73) Patentinhaber: MG Technologies AG, 60325 Frankfurt am Main (DE)
(72) Erfinder: BIRKE, Gerhard, D-60389 Frankfurt am Main (DE); HIRSCH, Martin, D-61381 Friedrichsdorf (DE); FRANZ, Volker, D-60486 Frankfurt am Main (DE)
(74) Vertreter: Revesz, Veronika
(86) Internationale Anmeldenummer: EP9901158
(87) Internationale Veröffentlichungsnummer: WO99048583

(56) Entgegenhaltungen:
- EP-A- 0 467 441
- GB-A- 988 084
- US-A- 3 791 110

## Beschreibung

Die Erfindung betrifft ein Verfahren zum Abtrennen von in einem Gasstrom dampfförmig enthaltenem Phthalsäureanhydrid (PSA) durch Kühlen des Gasstroms und verfestigen des PSA in einem Kühler, der ein Wirbelbett enthält, welches aus PSA enthaltendem Granulat besteht, wobei das Wirbelbett indirekt gekühlt wird.

Ein solches Verfahren ist aus GB-A-988084 bekannt. Bei diesem Verfahren leitet man den PSA-haltigen Gasstrom in den unteren Bereich eines gekühlten Wirbelbettes. Hierbei wird zwangsläufig das Gas jedoch weitgehend in Form von Blasen durch das Wirbelbett geführt, wobei sich die Blasen sehr stabil verhalten. Die Gasblasen verhindern einen intensiven Wärmeübergang zwischen dem in den Blasen enthaltenen PSA-Dampf und den relativ kalten Feststoffpartikeln des Wirbelbettes. Die Kühlung des PSA-Dampfes bleibt deshalb mangelhaft und man erreicht erfahrungsgemäß nur, daß höchstens 50% des PSA-Dampfes im Wirbelbett kondensiert werden.

Der Erfindung liegt die Aufgabe zugrunde, mit Hilfe eines indirekt gekühlten Wirbelbettes den dampfförmiges PSA enthaltenden Gasstrom intensiv zu kühlen, so daß das PSA aus dem Gasstrom mit hoher Wirksamkeit abgeschieden wird.
Erfindungsgemäß wird dies dadurch erreicht, daß man den dampfförmiges PSA enthaltenden Gasstrom durch ein im unteren Bereich des Kühlers angeordnetes vertikales Rohr aufwärts leitet, daß das vertikale Rohr ein oberes Mündungsende aufweist, daß das Rohr und sein Mündungsende vom indirekt gekühlten Wirbelbett umgeben sind, dessen Temperatur im Bereich von 20 bis 90°C liegt und dem man von unten Fluidisierungsgas zuführt,
wobei die Suspensionsdichte des Wirbelbettes im Bereich von 300 bis 700 kg/m³ liegt, daß der Innenbereich des Rohrs kein Wirbelbett aufweist, daß vom Wirbelbett über das Mündungsende des vertikalen Rohrs ständig Granulat aus dem Wirbelbett in den dampfförmiges PSA enthaltenden Gasstrom eingetragen und vom Gasstrom zu einem oberhalb des Rohrs und über dem Wirbelbett im Kühler befindlichen Beruhigungsraum mitgeführt wird, wobei im Gasstrom enthaltenes dampfförmiges PSA gekühlt und verfestigt wird und wobei verfestigtes PSA mindestens teilweise aus dem Beruhigungsraum auf das Wirbelbett fällt, daß man Gas aus dem Beruhigungsraum und aus dem Kühler abführt und daß man PSA enthaltendes Granulat aus dem Wirbelbett abzieht.

Durch das erfindungsgemäße Verfahren werden im Kühler über 90 % des eingeleiteten PSA-Dampfes gekühlt und verfestigt. Üblicherweise wird man vom Wirbelbett im Bereich des Mündungsendes des vertikalen Rohrs 10 bis 50 kg Feststoff pro Nm³ Gas in den PSA enthaltenden Gasstrom einbringen. Der Beruhigungsraum und auch der Bereich direkt oberhalb des Mündungsendes des vertikalen Rohrs ist frei von einem Wirbelbett. Es befinden sich dort nur relativ geringe Feststoffmengen, so daß sich dort auch keine Gasblasen bilden können, wie sie in einem Wirbelbett aber unvermeidbar sind.

Es ist vorteilhaft, wenn das Granulat des Wirbelbettes zu mindestens 80 Gew.% aus Korngrößen von höchstens 1 mm besteht, wenn man ohne Hilfsgranulat arbeitet. Das relativ feinkörnige Granulat ist gut fließfähig und kann im Wirbelbett mit hohen Wärmeübergangszahlen indirekt gekühlt werden.

Zum Fluidisieren des Wirbelbettes können verschiedenartige Gase verwendet werden. Zweckmäßigerweise verwendet man aus dem Kühler abgezogenes, mindestens teilweise entstaubtes Gas oder aber Luft oder ein Gemisch dieser beiden Gase.

Der das dampfförmige PSA enthaltende Gasstrom kommt üblicherweise aus einem Reaktor zum katalytischen Erzeugen von PSA aus Orthoxylol oder Naphthalin mit Luft. Der dampfförmiges PSA enthaltende Gasstrom, der auf diese bekannte Weise erzeugt wird, kann zunächst in einem Abhitzekessel ein- oder mehrstufig indirekt gekühlt werden, bevor man ihn zur Schlußkühlung in das vertikale Rohr leitet. Eine Vorkühlung ohne Kondensation und ohne Erzeugen von festem PSA kann vorteilhaft sein, wenn man die Wärmebelastung in der Schlußkühlung verringern will.

Ein Kühler der Art, wie er beim erfindungsgemäßen Verfahren zum Verfestigen des PSA-Dampfes verwendet wird, ist in EP-B-0467441 beschrieben. Dieser Kühler ist insbesondere zur Kühlung eines Abgases aus der Verhüttung von Bleierz vorgesehen, wobei die Gasreinigung vor allem den Erfordernissen des Umweltschutzes gerecht wird. Es hat sich nun gezeigt, daß der prinzipiell bekannte Kühler in der Lage ist, relativ große Mengen an PSA, die dampfförmig herangeführt werden, zu verfestigen.

Für den Aufbau des Wirbelbettes im Kühler, welches das vertikale Rohr umgibt, kann man ohne ein Hilfsgranulat oder aber mit einem solchen Hilfsgranulat, z.B. Sand mit Körnungen von etwa 0,05 bis 1 mm, arbeiten. Im gekühlten Wirbelbett kondensiert PSA auf dem Hilfsgranulat und wird mit diesem abgezogen. Außerhalb des Wirbelbettes trennt man das rohe PSA vom Hilfsgranulat, z.B. durch Abschmelzen, und kann das Hilfsgranulat wieder zurück in das Wirbelbett führen. Wenn man ohne Hilfsgranulat arbeitet, entfällt dieser Trennungsschritt.

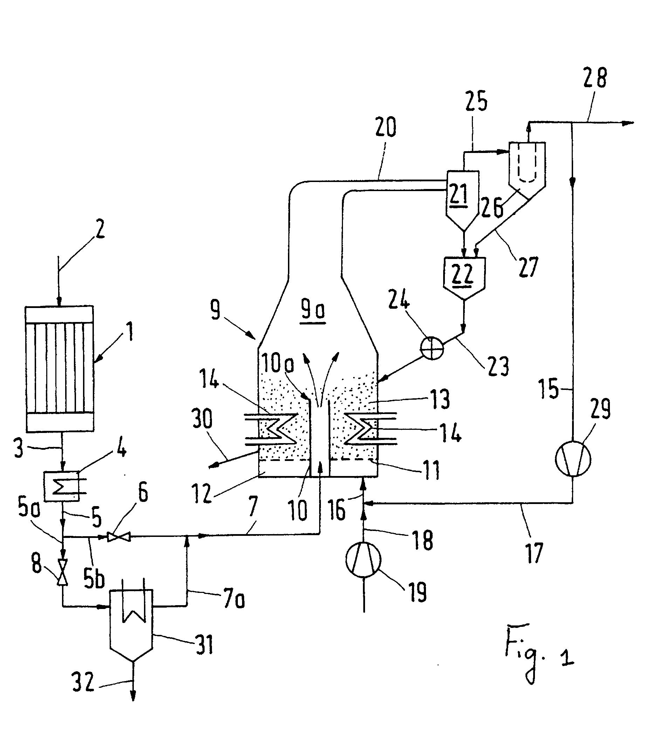

Ausgestaltungsmöglichkeiten des Verfahrens werden mit Hilfe der Zeichnung erläutert. Die Zeichnung zeigt ein Fließschema des Verfahrens.

In an sich bekannter Weise wird im Röhrenreaktor (1) aus einem Gemisch von Naphthalin oder Orthoxylol und Luft, das in der Leitung (2) herangeführt wird, katalytisch PSA bei Temperaturen von etwa 300 bis 500°C erzeugt. Der dampfförmiges PSA enthaltende Gasstrom, der als Produkt der Umsetzung im Reaktor 1 entsteht, strömt in der Leitung (3) zu einem Abhitzekessel (4), in welchem eine erste Kühlung stattfindet. Dabei kondensiert PSA noch nicht aus. Mit Temperaturen von üblicherweise 150 bis 250°C strömt der PSA-haltige Gasstrom dann durch die Leitungen (5) und (5b), durch das geöffnete Ventil (6) und die Leitung (7) zum Schlußkühler (9). Bei dieser Verfahrensvariante ist das Ventil (8) geschlossen.

Der Kühler (9) weist im unteren zentralen Bereich ein vertikales Rohr (10) auf, dazu einen Gasverteiler (11) und darunter eine Verteilkammer (12) für Fluidisierungsgas. Der das Rohr (10) umgebende Ringraum ist mit Kühlelementen (14) versehen, die zur Wärmeabfuhr von einem Kühlfluid durchströmt werden. Als Kühlfluid eignet sich z.B. Wasser oder Öl. Im Kühler (9) befindet sich im Ringraum über dem Gasverteiler (11) ein Wirbelbett (13) aus PSA-haltigem Granulat, wobei das Wirbelbett ein wenig über das obere Mündungsende (10a) des Rohrs (10) hinaus reicht. Mindestens 80 Gewichtsprozent des Granulats des Wirbelbettes weisen Korngrößen im Bereich von höchstens 1 mm auf. Fluidisierungsgas, das durch den Gasverteiler (11) aufwärts strömt, wird zuvor durch die Leitung (16) in die Verteilkammer (12) geführt. Bei diesem Fluidisierungsgas kann es sich entweder um rückgeführtes Gas aus der Leitung (17) oder um Luft aus der Leitung (18) handeln, die durch das Gebläse (19) angesaugt wird, oder um ein Gemisch aus Luft und rückgeführtem Gas.

Im Schlußkühler (9) liegt die Leerrohrgeschwindigkeit des Fluidisierungsgases im Wirbelbett (13) üblicherweise im Bereich von 0,1 bis 0,6 m/s. Die Gasgeschwindigkeiten im Rohr (10) liegen etwa im Bereich von 20 bis 50 m/s und im Beruhigungsraum (9a), der sich oberhalb des Mündungsendes (10a) befindet, ist die effektive Gasgeschwindigkeit etwa 2 bis 3 m/s. Das Volumen des Fluidisierungsgases beträgt 10 bis 30 % und zumeist 15 bis 25 % des Volumens des Gasstroms der Leitung (7). Die Suspensionsdichte im Wirbelbett (13) liegt im Bereich von 300 bis 700 kg/m³ und zumeist 350 bis 600 kg/m³. Das Wirbelbett (13) endet knapp oberhalb des Mündungsendes (10a) des Rohrs (10). Ein Wirbelbett befindet sich im Beruhigungsraum (9a) nicht. Auf diese Weise sorgt man dafür, daß 10 bis 50 kg Feststoff pro Nm³ Gas in den Gasstrom eingebracht werden, der im Rohr (10) aufwärts strömt. Im Wirbelbett (13) gekühltes Granulat mischt sich auf diese Weise mit dem das Rohr (10) verlassenden Gasstrom, wird von diesem aufwärts in den Beruhigungsraum (9a) mitgeführt und sorgt dadurch für eine schnelle und intensive Abkühlung des Gases und des dampfförmigen PSA. In den Beruhigungsraum (9a) wird eine Gas-Feststoff-Suspension geblasen, wobei die Gasgeschwindigkeit durch die Expansion des Gasstrahls rasch abfällt. Hierbei verlieren die Feststoffe an Geschwindigkeit und fallen wieder in die Wirbelschicht (13) zurück. Es kann zweckmäßig sein, die Innenwand des Kühlers (9), auch im Bereich des Beruhigungsraumes (9a) und darüber, mit Kühlelementen zu versehen, was aber in der Zeichnung zur Vereinfachung nicht berücksichtigt wurde.

Um ausreichend gekühltes Granulat im Wirbelbett (13) bereitzuhalten, hält man die Temperaturen dort üblicherweise bei 20 bis 90°C und vorzugsweise 50-80°C. Als Produkt zieht man durch die Leitung (30) PSA-Granulat aus dem Kühler (9) ab. Dieses Granulat wird dann üblicherweise noch einer an sich bekannten Feinreinigung zugeführt, wie sie z.B. in DE-C-3538911 beschrieben ist. Wenn man mit einem Hilfsgranulat, z.B. Sand, arbeitet, werden dessen Körner im gekühlten Wirbelbett durch anhaftendes PSA vergrößert. Dieses Granulat zieht man in der Leitung (30) ab und trennt PSA vom Hilfsgranulat, z.B. durch Abschmelzen.

Gas, welches eine gewisse Menge an Feststoffen mitführt, wird vom oberen Ende des Kühlers (9) durch den Kanal (20) abgeleitet und zunächst zu einem Abscheidezyklon (21) geführt. Abgeschiedene Feststoffe gelangen in den Pufferbehälter (22) und von da durch die Leitung (23) und ein Dosierorgan (24) zurück in den Kühler (9). Das den Abscheider (21) verlassende Gas strömt durch die Leitung (25) zu einem Filter (26), wobei abgeschiedene Feststoffe durch die Leitung (27) dem Pufferbehälter (22) aufgegeben werden. Das Filter (26) kann z.B. ein Schlauch- oder Elektrofilter sein. Entstaubtes Gas zieht in der Leitung (28) ab und kann ganz oder teilweise zu einer nicht dargestellten Nachverbrennung geführt werden. Üblicherweise zweigt man einen Teilstrom des entstaubten Gases in der Leitung (15) ab und führt es durch das Gebläse (29) und die Leitung (17) als Fluidisierungsgas zurück in den Kühler (9).

Eine Verfahrensvariante besteht darin, das aus dem Abhitzekessel (4) kommende, PSA-haltige Gas bei geschlossenem Ventil (6) und geöffnetem Ventil (8) durch einen Kühler (31) zu führen, in welchem ein Teil des PSA kondensiert und flüssig in der Leitung (32) abgezogen wird. Das restliche, PSA-haltige Gas wird dann durch die Leitungen (7a) und (7) zum Schlußkühler (9) geführt.

### Beispiel:

Man arbeitet mit einer der Zeichnung entsprechenden Verfahrensführung, wobei das Ventil (6) geschlossen und das Ventil (8) offen ist. Der Schlußkühler (9) hat eine Gesamthöhe von 10 m, einen Durchmesser im Bereich des Wirbelbettes (13) von 3 m und eine Höhe zwischen dem Verteiler (11) und der oberen Mündung (10a) des Rohrs (10) von 2 m. Im Rohr (10), das einen Durchmesser von 0,7 m hat, beträgt die Gasgeschwindigkeit 40 m/sec, die Leerrohrgeschwindigkeit des Fluidisierungsgases im Ringraum (13) beträgt 0,3 m/sec. Das Wirbelbett (13) hat eine Suspensionsdichte von 450 kg/m³, die Korngrößen des PSA-Granulats liegen unter 1 mm und der Mittelwert d₅₀ beträgt 0,3 mm. Es wird ohne Hilfsgranulat im Kühler (9) gearbeitet.

Pro Stunde werden im Röhrenreaktor (1) 3900 kg Orthoxylol mit 51600 kg Luft umgesetzt. Die Drücke liegen zwischen 1 und 1,5 bar, die nachfolgend gegebenen Daten sind teilweise berechnet.

Bei den in der Tabelle genannten Gasen handelt es sich um O₂, N₂, CO₂ und H₂O enthaltende Gemische.

| **Leitung** | **3** | **7** | **30** |
|---|---|---|---|
| PSA (kg/h) | 4283 | 2364 | 2324 |
| Orthoxylol (kg/h) | 0,5 | 0,5 | -- |
| Nebenprodukte (kg/h) | 245 | 243 | 2 |
| Gase (kg/h) | 50990 | 50980 | 1 |
| Temperatur (°C) | 370 | 137 | 65 |

Pro Stunde werden in der Leitung (18) 2000 m³ Luft herangeführt, in der Leitung (17) strömen 6000 m³ wasserdampfhaltiges Gas mit 100°C. Durch die Leitung (28) werden 50300 m³ wasserdampfhaltiges Gas aus dem Verfahren entfernt.

## Patentansprüche

1. Verfahren zum Abtrennen von in einem Gasstrom dampfförmig enthaltenem Phthalsäureanhydrid (PSA) durch Kühlen des Gasstroms und Verfestigen des PSA in einem Kühler, der ein Wirbelbett enthält, welches aus PSA enthaltendem Granulat besteht, wobei das Wirbelbett indirekt gekühlt wird, **dadurch gekennzeichnet, daß** man den dampfförmiges PSA enthaltenden Gasstrom durch ein im unteren Bereich des Kühlers angeordnetes vertikales Rohr aufwärts leitet, daß das vertikale Rohr ein oberes Mündungsende aufweist, daß das Rohr und sein Mündungsende vom indirekt gekühlten Wirbelbett umgeben sind, dessen Temperatur im Bereich von 20 bis 90°C liegt und dem man von unten Fluidisierungsgas zuführt, wobei die Suspensionsdichte des Wirbelbettes im Bereich von 300 bis 700 kg/m³ liegt, daß der Innenbereich des Rohrs kein Wirbelbett aufweist, daß vom Wirbelbett über das Mündungsende des vertikalen Rohrs ständig Granulat aus dem Wirbelbett in den dampfförmiges PSA enthaltenden Gasstrom eingetragen und vom Gasstrom zu einem oberhalb des Rohrs und über dem Wirbelbett im Kühler befindlichen Beruhigungsraum mitgeführt wird, wobei im Gasstrom enthaltenes dampfförmiges PSA gekühlt und verfestigt wird und wobei verfestigtes PSA mindestens teilweise aus dem Beruhigungsraum auf das Wirbelbett fällt, daß man Gas aus dem Beruhigungsraum und aus dem Kühler abführt und daß man PSA enthaltendes Granulat aus dem Wirbelbett abzieht.

2. Verfahren nach Anspruch 1, **dadurch gekennzeichnet, daß** man vom Wirbelbett im Bereich des Mündungsendes des vertikalen Rohrs 10 bis 50 kg Feststoff pro Nm³ Gas in den PSA enthaltenden Gasstrom einbringt.

3. Verfahren nach Anspruch 1 oder 2, **dadurch gekennzeichnet, daß** der dampfförmiges PSA enthaltende Gasstrom ein- oder mehrstufig indirekt gekühlt wird, bevor man ihn in das vertikale Rohr des Kühlers leitet.

4. Verfahren nach Anspruch 1, **dadurch gekennzeichnet, daß** das aus dem Kühler abgeführte Gas nach Abtrennen von Feststoffen mindestens teilweise als Fluidisierungsgas in das Wirbelbett geleitet wird.

5. Verfahren nach Anspruch 1 oder einem der folgenden, **dadurch gekennzeichnet, daß** das Granulat des Wirbelbettes zu mindestens 80 Gewichtsprozent Korngrößen von höchstens 1 mm aufweist.

6. Verfahren nach Anspruch 1 oder einem der folgenden, **dadurch gekennzeichnet, daß** man Luft als Fluidisierungsgas in das Wirbelbett leitet.

7. Verfahren nach Anspruch 1 oder einem der folgenden, **dadurch gekennzeichnet, daß** man den dampfförmiges PSA enthaltenden Gasstrom zum Kühlen zunächst durch einen indirekten Wärmeaustauscher und anschließend in das vertikale Rohr des Kühlers leitet, wobei man aus dem indirekten Wärmeaustauscher flüssiges PSA abzieht.

8. Verfahren nach Anspruch 1 oder einem der folgenden, **dadurch gekennzeichnet, daß** das Wirbelbett ein Hilfsgranulat enthält.

## Claims

1. A process of separating phthalic acid anhydride (PA) contained in the form of vapour in a gas stream by cooling the gas stream and solidifying the PA in a cooler which contains a fluidised bed that consists of granules containing PA, wherein the fluidised bed is cooled indirectly, **characterised in that** the gas stream containing vaporous PA is passed upwards through a vertical tube disposed in the lower portion of the cooler, that the vertical tube has an upper orifice, that the tube and its orifice are surrounded by the indirectly cooled fluidised bed, whose temperature lies in the range from 20 to 90°C and to which fluidising gas is supplied from the bottom, the suspension density of the fluidised bed lying in the range from 300 to 700 kg/m³, that the inner portion of the tube has no fluidised bed, that from the fluidised bed through the orifice of the vertical tube granules for the fluidised bed are constantly introduced into the gas stream containing vaporous PA and are entrained by the gas stream to a settling space disposed in the cooler upstream of the tube and above the fluidised bed, wherein vaporous PA contained in the gas stream is cooled and solidified, and wherein solidified PA at least partly drops from the settling space onto the fluidised bed, that gas is withdrawn from the settling space and from the cooler, and that granules containing PA are withdrawn from the fluidised bed.

2. A process according to Claim 1, **characterised in that** 10 to 50 kg solids per sm³ gas are introduced into the gas stream containing PA from the fluidised bed in the vicinity of the orifice of the vertical tube.

3. A process according to Claim 1 or 2, **characterised in that** the gas stream containing vaporous PA is cooled indirectly in one or more stages, before it is introduced into the vertical tube of the cooler.

4. A process according to Claim 1, **characterised in that** upon separating solids, the gas withdrawn from the cooler is at least partly introduced as fluidising gas into the fluidised bed.

5. A process according to Claim 1 or any of the following claims, **characterised in that** the granules of the fluidised bed comprise at least 80 wt-% grain sizes of not more than 1 mm.

6. A process according to Claim 1 or any of the following claims, **characterised in that** air is introduced into the fluidised bed as fluidising gas.

7. A process according to Claim 1 or any of the following claims, **characterised in that** for cooling purposes the gas stream containing vaporous PA is first of all passed through an indirect heat exchanger and then into the vertical tube of the cooler, where liquid PA is withdrawn from the indirect heat exchanger.

8. A process according to Claim 1 or any of the following claims, **characterised in that** the fluidised bed contains auxiliary granules.

## Revendications

1. Procédé de séparation d'anhydride d'acide phtalique (AAS) contenu sous forme de vapeur dans un courant de gaz, par refroidissement du courant de gaz et solidification de l'AAS dans un dispositif de refroidissement qui comporte un lit fluidisé constitué d'un granulé contenant de l'AAS, le lit fluidisé étant refroidi par voie indirecte, **caractérisé en ce que** l'on envoie le courant de gaz contenant de l'AAS sous forme de vapeur en mouvement ascendant dans un tuyau vertical disposé à la partie inférieure du dispositif de refroidissement, **en ce que** le tuyau vertical a une extrémité supérieure d'embouchure, **en ce que** le tuyau et son extrémité d'embouchure sont entourés par le lit fluidisé refroidi par voie indirecte dont la température est de l'ordre de 20 à 90°C et auquel on envoie par le bas du gaz de fluidisation, la masse volumique de suspension du lit fluidisé étant de l'ordre de 300 à 700 kg/m³, **en ce que** la partie intérieure du tuyau n'a pas de lit fluidisé, **en ce que** l'on introduit, par le lit fluidisé, par l'extrémité d'embouchure du tuyau vertical, constamment du granulé provenant du lit fluidisé dans le courant de gaz contenant de l'AAS sous forme de vapeur et on l'entraîne par le courant de gaz vers une chambre de tranquillisation, se trouvant au-dessus du tuyau et sur le lit fluidisé, dans le dispositif de refroidissement, de l'AAS sous forme de vapeur contenu dans le courant de gaz étant refroidi et solidifié et l'AAS solidifié précipitant au moins en partie dans la chambre de tranquillisation sur le lit fluidisé, **en ce que** l'on évacue du gaz de la chambre de tranquillisation et du dispositif de refroidissement et que l'on retire du granulé contenant de l'AAS du lit fluidisé.

2. Procédé suivant la revendication 1, **caractérisé en ce que** l'on introduit par le lit fluidisé, dans la partie de l'extrémité de l'embouchure du tuyau vertical, de 10 à 50 kg de matière solide par m³ de gaz pris dans les conditions normales de température et de pression dans le courant de gaz contenant de l'AAS.

3. Procédé suivant la revendication 1 ou 2, **caractérisé en ce que** l'on refroidit par voie indirecte, en un étage ou en plusieurs étages, le courant de gaz contenant de l'AAS sous forme de vapeur avant de l'envoyer dans le tuyau vertical du dispositif de refroidissement.

4. Procédé suivant la revendication 1, **caractérisé en ce que** l'on envoie le gaz évacué du dispositif de refroidissement après en avoir séparé les matières solides au moins en partie en tant que gaz de fluidisation au lit fluidisé.

5. Procédé suivant la revendication 1 ou l'une des suivantes, **caractérisé en ce que** le granulé du lit fluidisé présente pour au moins 80 pour-cent en poids des granulométries d'au plus 1 mm.

6. Procédé suivant la revendication 1 ou l'une des suivantes, **caractérisé en ce que** l'on envoie de l'air comme gaz de fluidisation au lit fluidisé.

7. Procédé suivant la revendication 1 ou l'une des suivantes, **caractérisé en ce que** l'on envoie le courant de gaz contenant de l'AAS sous forme de vapeur en vue de le refroidir, d'abord dans un échangeur de chaleur par voie indirecte, et ensuite dans le tuyau vertical du dispositif de refroidissement, en soutirant de l'AAS liquide de l'échangeur de chaleur par voie indirecte.

8. Procédé suivant la revendication 1 ou l'une des suivantes, **caractérisé en ce que** le lit fluidisé comporte un granulé auxiliaire.
